# FASCICULE DE BREVET EUROPEEN

(11) **EP 0 745 376 B1**
(45) Date de publication et mention de la délivrance du brevet: **11.03.1998**
(21) Numéro de dépôt: 96401024.3
(22) Date de dépôt: 10.05.1996
(51) Int. Cl.: A61K 7/48, A61K 7/02, A61K 7/025, A61K 7/027

(54) **Composition cosmétique sous forme de pâte souple et son procédé de préparation**
Kosmetische Zubereitung in weicher Pastenform und sein Verfahren zur Herstellung
Cosmetic composition in soft paste form and process for the preparation thereof

(30) Priorité: 01.06.1995 FR 9506537; 26.07.1995 FR 9509107
(43) Date de publication de la demande: 04.12.1996
(73) Titulaire: L'OREAL, 75008 Paris (FR)
(72) Inventeur: Roulier, Véronique, 75002 Paris (FR); Daubige, Thérèse, 77480 Bray sur Seine (FR)
(74) Mandataire: Dodin, Catherine

(56) Documents cités:
- EP-A- 0 524 892
- EP-A- 0 530 084
- EP-A- 0 530 085
- EP-A- 0 655 234
- CHEMICAL ABSTRACTS, vol. 104, no. 2, 13 Janvier 1986 Columbus, Ohio, US; abstract no. 10386k, page 301; colonne G; XP002011900 & DD-A-221 078 (VEB KOSMETIK KOMBINAT)

## Description

La présente invention a trait à une composition cosmétique se présentant sous forme d'une pâte souple, pouvant être utilisée pour le soin et/ou le maquillage des lèvres et/ou de la peau.

Les compositions cosmétiques pouvant être appliquées sur la peau ou les lèvres comme produit de maquillage ou de soin, telles que les bases pour lèvres ou les rouges à lèvres par exemple, contiennent généralement des corps gras dont des cires, des pigments et/ou charges et, éventuellement, des additifs. Il est connu que plus la quantité de cires présente dans la composition est importante, plus celle-ci a une consistance ferme, ce qui permet son utilisation sous forme de bâton. Or la présentation, en particulier d'un rouge à lèvres, sous forme de bâton présente certains inconvénients: le dessin des contours des lèvres est malaisé et la tenue du bâton à la chaleur n'est pas optimale.
Il est également connu des compositions cosmétiques se présentant sous forme de pâte souple, applicables à l'aide d'un pinceau, par exemple. Ces compositions contiennent peu ou pas de cires, ce qui permet de les prélever et de les appliquer aisément, étant donné qu'une quantité importante de cires conduirait à une composition de viscosité plus élevée qui serait alors inapplicable.
Toutefois, les cires peuvent se révéler des composés intéressants cosmétiquement. En particulier, les cires ayant une température de fusion commençante élevée, c'est-à-dire supérieure à environ 100°C, peuvent apporter certaines propriétés telles que douceur et glissant lors de l'application, ainsi qu'une texture non collante. Elles peuvent également conférer à la composition des qualités de consistance, d'onctuosité et de tenue du film appliqué remarquables. Or, un problème se pose lorsque l'on essaie d'introduire ces cires, ou d'autres composés possédant une température de fusion commençante élevée, dans une composition comprenant par ailleurs d'autres corps gras tels que les cires ou les huiles couramment utilisées en cosmétique. En effet, lorsque l'on chauffe la totalité du mélange à une température à laquelle les cires à fusion élevée fondent, on observe une dénaturation des autres corps gras présents dans la formule, dénaturation qui consiste en une oxydation desdits autres corps gras. Il n'est ainsi pas possible d'obtenir ainsi une composition de bonne qualité.
Afin d'éviter cette dénaturation des autres corps gras, on peut chauffer, dans un premier temps, les cires à fusion élevée à une température à laquelle elles fondent, puis introduire, dans un second temps, à une température moins élevée, les autres corps gras, cires et huiles. Dans ce cas, on assiste toutefois à une cristallisation des cires à fusion élevée lors de l'ajout des autres corps gras, d'où l'obtention d'un mélange non homogène et d'une composition finale présentant une texture granuleuse au toucher et/ou à l'application, incompatible avec une utilisation cosmétique satisfaisante.
Il n'est ainsi donc pas possible d'obtenir, par un procédé de l'art antérieur, une composition cosmétique comprenant une cire à fusion élevée et présentant des qualités cosmétiques correctes.
On connaît, par le document EP524892, un procédé de préparation d'une dispersion solide anhydre comprenant un corps gras dont une cire de point de fusion supérieur à 55°C, et un alcool polyhydrique, dans lequel on chauffe le corps gras et l'alcool polyhydrique à 65-95°C, et on mélange à l'aide d'une turbine ayant une vitesse de rotation supérieure à 1500 tours/min.
On connaît, par le document EP530084, une composition pour le maquillage des cils et sourcils, comprenant une phase dispersée qui comprend une charge et une cire hydrocarbonée, et une phase dispersante aqueuse qui comprend un polymère hydrosoluble.
On connaît également, par le document EP530085, une composition pour le maquillage de la peau comprenant une cire de silicone dispersée dans une phase aqueuse qui comprend deux polymères acryliques hydrosolubles.
On connaît encore, par le document Chemical Abstract, vol 104, no 2, abstract 10386k, page 301, des bases pour compositions cosmétiques comprenant un mélange de deux combinaisons de cires, la première combinaison comprenant une cire de Montan et une cire de polyéthylène, la seconde combinaison comprenant une cire de Montan et un copolymère polyéthylène.

La présente invention a pour but de permettre l'obtention d'une telle composition et propose un procédé permettant l'obtention d'une composition cosmétique, notamment anhydre, de texture homogène et cosmétiquement satisfaisante, tout en contenant des corps gras usuellement utilisés tels que des cires et/ou des huiles, d'une part, et des composés, notamment des cires, ayant une température de fusion commençante élevée, d'autre part.

Un objet de la présente invention est donc une composition extrudée, se présentant sous forme de pâte souple et ayant une viscosité dynamique à 25°C, de 3-30 Pa.s, mesurée avec un viscosimètre rotatif CONTRAVES TV équipé d'un mobile "MS-r4" à la fréquence de 60 Hz, comprenant une phase grasse et au moins un composé ayant une température de fusion commençante au moins égale à 100°C.
Un autre objet de l'invention est un procédé de préparation de cette composition, dans lequel, dans une première étape, on chauffe le composé ayant une température de fusion commençante au moins égale à environ 100°C, à une température T1 à laquelle il fond; dans une seconde étape, on ajoute et mélange au moins une première partie des autres corps gras à une température T2 à laquelle ils sont fondus sans être altérés, tout en maintenant un certain malaxage; puis on poursuit le malaxage du mélange pendant au moins une partie de son refroidissement jusqu'à température ambiante; les opérations de mélange et de malaxage étant effectuées dans au moins un mélangeur-extrudeur.

Par 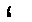 température de fusion commençante 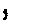 , on entend dans la présente description la température à laquelle le composé commence à fondre.

On peut notamment déterminer cette température par ATD (analyse thermique différentielle) qui permet l'obtention du thermogramme (ou courbe de fusion) du composé, notamment de la cire, considéré. La température de fusion commençante correspond à la température à laquelle on peut observer un changement de pente notable dans le thermogramme. Le point de fusion, quant à lui, représente le point minimum dudit thermogramme.
Dans la suite de la présente description, les termes 'composé ayant une température de fusion commençante au moins égale à environ 100°C' et 'composé à fusion élevée' seront utilisés de manière équivalente, afin de désigner les composés mis en oeuvre dans le cadre de la présente invention.

On a constaté que la composition selon l'invention peut comprendre une quantité importante de composés à fusion élevée, notamment de cires à fusion élevée, tout en restant cosmétiquement satisfaisante. Ladite composition présente de plus une texture peu collante, un bon glissant à l'application et une bonne tenue au cours du temps.

La composition selon la présente invention est donc une pâte souple dont on peut mesurer la viscosité, par opposition à la structure solide d'un bâton, ou stick, dont on ne peut pas mesurer la viscosité. Ladite viscosité dynamique à 25°C est généralement comprise entre 3 et 30 Pa.s, mesurée avec un viscosimètre rotatif CONTRAVES TV équipé d'un mobile "MS-r4" à la fréquence de 60 Hz.

La composition selon l'invention comprend donc au moins un composé à fusion élevée, dont la température de fusion commençante est au moins égale à environ 100°C, de préférence supérieure à environ 110°C.
Parmi ces composés, on peut citer les cires, les polymères et les matières plastiques, ainsi que leurs mélanges.
Parmi les cires, dont la température de fusion est généralement inférieure à 150°C, on peut citer certaines cires de polyéthylène, telles que la cire 'Polywax 2000T-6' de Petrolite (température de fusion commençante : 125°C), la cire 'PED 191' de Hoechst (120°C) et la cire 'Epolene N-14' de Eastman Kodak (106°C), seule ou en mélange.

Ces cires à fusion élevée peuvent être les seules cires présentes dans la composition, les autres corps gras pouvant être des huiles, des gommes et/ou des composés pâteux. Elles peuvent également être associées à d'autres cires dont la température de fusion commençante est, par exemple, de l'ordre de 40-70°C. Parmi les polymères et les matières plastiques, dont la température de fusion est généralement supérieure à 150°C, et de préférence inférieure à 300°C, on peut citer les polypropylènes, les polyéthylènes, les PVC, les polymères vinyliques ou acryliques tels que le polystyrène, les polyamides tels que le Nylon, et leurs mélanges. Ces polymères ou matières plastiques peuvent éventuellement se présenter sous forme de poudre ou micronisée.
Les composés à fusion élevée peuvent être présents dans la composition selon l'invention à raison de 2-50% en poids, de préférence 10-30%.

La composition selon l'invention peut donc en outre comprendre les corps gras usuellement utilisés en cosmétique, notamment des huiles, gommes et/ou cires de silicone, et/ou des huiles et/ou cires végétales, minérales, animales et/ou synthétiques, éventuellement volatiles. Ces autres corps gras, ainsi que leurs quantités, pourront être choisis sans problème par l'homme du métier sur base de ses connaissances générales, de manière à obtenir une composition ayant les propriétés/qualités souhaitées. D'une manière générale, le reste de la phase grasse peut représenter 50-98% en poids de la composition, de préférence 70-90% en poids.
Parmi les cires susceptibles d'être présentes dans la composition selon l'invention, on peut citer les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de montan; les ozokérites; les cires de polyéthylène; les cires obtenues par synthèse de Fischer-Tropsch; les huiles hydrogénés, les esters gras et les glycérides concrets à 25°C. On peut également utiliser des cires de silicone, parmi lesquelles on peut citer les alkyls, alcoxys et/ou esters de polyméthylsiloxane.
Parmi les huiles susceptibles d'être présentes dans la composition selon l'invention, on peut citer les huiles hydrocarbonées telles que l'huile de paraffine ou de vaseline; le perhydrosqualène; l'huile d'Arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane ou les isoparaffines.

La composition peut également comprendre une phase pulvérulente, généralement présente à raison de 0-40 % en poids, de préférence 10-25% en poids, et qui peut comprendre des pigments et/ou des nacres et/ou des charges habituellement utilisés dans les compositions cosmétiques.
Les pigments peuvent être présents dans la composition à raison de 0-30% en poids de la composition finale, et de préférence à raison de 5-20%. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Les nacres peuvent être présentes dans la composition à raison de 0-20% en poids, de préférence à un taux élevé de l'ordre de 5-15% en poids. Parmi les nacres envisageables, on peut citer le mica recouvert d'oxyde de titane, d'oxyde de fer, de pigment naturel ou d'oxychlorure de bismuth ainsi que le mica titane coloré.
Les charges, qui peuvent être présentes à raison de 0-40% en poids, de préférence 10-25%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères creuses telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit de soin ou de maquillage de la peau, en particulier sous la forme d'un fond de teint, d'un fard à joues ou à paupières, d'un rouge à lèvres ou d'une base de soin pour les lèvres, ou encore d'une crème de soin pour le corps ou le visage.

Afin de préparer la composition selon l'invention, dans une première étape, on peut chauffer le composé à fusion élevée à une température T1 à laquelle il est fondu, tout en maintenant un certain malaxage en continu.
On peut éventuellement ajouter en mélange tout ou partie des autres constituants de la composition, autres que les corps gras, qui peuvent être chauffés sans altération à cette température T1.
Dans une seconde étape, on ajoute et mélange au moins une première partie des corps gras, à une température T2, à laquelle ils sont fondus sans être altérés, tout en maintenant un certain malaxage,
De préférence, T2 est de l'ordre de la température de fusion de ces corps gras, et est inférieure à T1.
On peut également ajouter tout ou partie des constituants autres que les corps gras lors de cette seconde étape.
Cette addition des corps gras et des autres constituants peut être effectué en une ou plusieurs étapes. Ainsi, on peut prévoir une 3ème, voire une 4ème étape, semblables à la seconde étape, dans lesquelles on ajoute à T3, respectivement T4, les corps gras qui ont une température de fusion proche, éventuellement avec les autres constituants tels que charges ou pigments.

Enfin, on poursuit le malaxage du mélange final pendant au moins une partie de son refroidissement jusqu'à température ambiante.
Le procédé selon l'invention est caractérisé par le fait que les opérations de mélange et de malaxage étant effectuées dans au moins un mélangeur-extrudeur.
Les opérations de mélange et de malaxage sont effectuées dans au moins un extrudeur.
En effet, on a constaté que l'utilisation d'au moins un mélangeur-extrudeur permet d'obtenir une pâte de qualité très constante de façon reproductible. De plus, il est possible, en adaptant la filière de sortie du mélangeur-extrudeur, de conditionner la composition en ligne à la sortie de ladite filière.
Il est préférable d'effectuer également les opérations de chauffage dans un mélangeur-extrudeur.
Il est également possible d'effectuer les opérations de chauffage, de mélange et de malaxage dans plusieurs extrudeurs, qui peuvent être disposés à la suite les uns des autres.
Toutefois, on utilise de préférence un extrudeur bi-vis unique pour effectuer la totalité du procédé.
Les conditions dans lesquelles l'extrusion peut être effectuée sont décrites dans la demande de brevet FR94-00756 dont le contenu est incorporé à la présente demande par référence.
Ce procédé permet d'obtenir une composition se présentant sous forme de pâte souple et homogène, bien qu'elle contienne des composés à fusion élevée, éventuellement en quantité importante. De plus, la composition obtenue après extrusion présente une douceur particulière, et procure une certaine sensation de glissant lorsqu'elle est appliquée sur la peau, tout en évitant l'aspect et la sensation de gras huileux.

L'invention est illustrée plus en détail dans les exemples suivants.

### Exemple 1

On prépare une base de soin ayant la composition suivante :

| | |
|---|---|
| .huile de sésame | 35 g |
| .lanoline | 35 g |
| .cire 'Epolene N-14' de Kodak | 15 g |
| .talc | 15 g |

La composition est préparée de la manière suivante : on introduit la cire 'Epolene N-14' dans la première partie d'un extrudeur bi-vis, qui est chauffée de telle manière que la cire fond (environ 130°C). On introduit les autres constituants dans une partie suivante, à une température de l'ordre de 85-90°C.
La température de sortie est de 30°C, la vitesse des vis de 400 tours/min et le temps de séjour d'environ 2 minutes.
On obtient en sortie une pâte souple de viscosité environ égale à 9 Pa.s, se présentant sous forme d'une seule phase stable et homogène, et pouvant être prélevée à l'aide d'un pinceau pour son application.

Cette composition permet l'obtention d'un film homogène, facilement applicable et s'étalant facilement et uniformément.
Cette composition présente un toucher glissant et non collant, et n'est absolument pas de texture granuleuse.

### Exemple 2

On prépare un rouge à lèvres ayant la composition suivante :

| | |
|---|---|
| .huile de sésame | 45 g |
| .lanolate d'isopropyle | 20 g |
| .cire 'Polywax 2000T-6' de Petrolite | 23 g |
| .pigments et charges | 12 g |

La composition est préparée selon l'exemple 1.

On obtient une pâte souple anhydre, de viscosité de 12 Pa.s, de texture peu collante, présentant un bon glissant à l'application et une bonne tenue dans le temps.

### Exemple 3

On prépare une base de soin ayant la composition suivante :

| | |
|---|---|
| .huile de sésame | 35 g |
| .lanoline | 35 g |
| .cire 'Epolene N-14' de Kodak | 15 g |
| .talc | 15 g |

La composition est mélangée à 150°C à l'aide d'une turbine de type Moritz et refroidie. On obtient une pâte hétérogène et de texture granuleuse.

## Revendications

1. Composition extrudée, se présentant sous forme de pâte souple et ayant une viscosité dynamique à 25°C, de 3-30 Pa.s, mesurée avec un viscosimètre rotatif CONTRAVES TV équipé d'un mobile "MS-r4" à la fréquence de 60 Hz, comprenant une phase grasse et 2-50% en poids d'au moins un composé ayant une température de fusion commençante au moins égale à 100°C, choisi parmi les cires, les polymères, les matières plastiques et leurs mélanges.

2. Composition selon la revendication 1, dans laquelle ledit composé est une cire ayant une température de fusion commençante supérieure à environ 110°C.

3. Composition selon l'une des revendications précédentes, dans laquelle ledit composé comprend au moins une cire de polyéthylène.

4. Composition selon l'une des revendications précédentes, dans laquelle ledit composé est choisi parmi la cire de polyéthylène 'Polywax 2000T-6' de Petrolite, la cire de polyéthylène 'PED 191' de Hoechst, la cire de polyéthylène 'Epolene N-14' de Eastman Kodak, et leurs mélanges.

5. Composition selon la revendication 1, dans laquelle ledit composé est un polymère ou une matière plastique choisis parmi les polypropylènes, les polyéthylènes, les PVC, les polymères vinyliques ou acryliques tels que le polystyrène, les polyamides tels que le Nylon, et leurs mélanges.

6. Composition selon la revendication 5, dans laquelle les polymères ou matières plastiques se présentent sous forme de poudre ou sous forme micronisée.

7. Composition selon l'une des revendications précédentes, dans laquelle le composé ayant une température de fusion commençante au moins égale à environ 100°C est présent à raison de 10-30% en poids, dans la composition.

8. Composition selon l'une des revendications précédentes, comprenant en outre des corps gras choisis parmi les huiles, es gommes, les cires et/ou les composés pâteux, hydrocarbonés et/ou siliconés, éventuellement volatils.

9. Composition selon la revendication 8, dans laquelle les corps gras représentent 50-98% en poids de la composition, de préférence 70-90% en poids.

10. Composition selon l'une des revendications 1 à 4 dans laquelle la cire ayant une température de fusion commençante au moins égale à environ 100°C est la seule cire présente dans la composition.

11. Composition selon l'une des revendications précédentes, comprenant une phase pulvérulente en une quantité de 0-40 % en poids, de préférence 10-25% en poids.

12. Composition selon l'une des revendications précédentes, se présentant sous la forme d'une composition cosmétique, et notamment sous la forme d'un fond de teint, d'un fard à joues ou à paupières, d'un rouge à lèvres ou d'une base de soin pour les lèvres, d'une crème de soin pour le corps ou le visage.

13. Procédé de préparation d'une composition selon l'une des revendications précédentes, dans lequel :
. dans une première étape, on chauffe le composé ayant une température de fusion commençante au moins égale à environ 100°C, à une température T1 à laquelle il fond,
. dans une seconde étape, on ajoute et mélange au moins une première partie des autres corps gras à une température T2 à laquelle ils sont fondus sans être altérés, tout en maintenant un certain malaxage,
. on poursuit le malaxage du mélange pendant au moins une partie de son refroidissement jusqu'à température ambiante,
les opérations de mélange et de malaxage étant effectuées dans au moins un mélangeur-extrudeur.

14. Procédé selon la revendication 13, dans lequel on ajoute tout ou partie des constituants autres que les corps gras lors de la première et/ou de la seconde étape.

15. Procédé selon l'une des revendications 13 à 14, dans lequel les opérations de chauffage sont effectuées dans un mélangeur-extrudeur.

16. Procédé selon l'une des revendications 13 à 15, dans lequel les opérations de chauffage, de mélange et de malaxage sont effectués dans plusieurs extrudeurs.

17. Procédé selon l'une des revendications 13 à 16, dans lequel les opérations de chauffage, de mélange et de malaxage sont effectués dans un extrudeur bi-vis unique.

## Claims

1. Extruded composition, provided in the form of a soft paste and having a dynamic viscosity at 25°C of 3-30 Pa.s, measured with a CONTRAVES TV rotational viscometer equipped with a rotor "MS-r4" at the frequency of 60 Hz, comprising a fatty phase and 2-50 % by weight of at least one compound having a temperature at onset of melting at least equal to about 100°C, chosen from waxes, polymers, plastics and mixtures thereof.

2. Composition according to Claim 1, in which the said compound is a wax having a temperature at onset of melting greater than about 110°C.

3. Composition according to one of the preceding claims, in which the said compound comprises at least one polyethylene wax.

4. Composition according to one of the preceding claims, in which the said compound is chosen from the polyethylene wax "Polywax 2000T-6" from Petrolite, the polyethylene wax "PED 191" from Hoechst, the polyethylene wax "Epolene N-14" from Eastman Kodak, and mixtures thereof.

5. Composition according to Claim 1, in which the said compound is a polymer or a plastic material chosen from polypropylenes, polyethylenes, PVCs, vinyl or acrylic polymers such as polystyrene, polyamides such as nylon, and mixtures thereof.

6. Composition according to Claim 5, in which the polymers or plastic materials are provided in powder form or in micronized form.

7. Composition according to one of the preceding claims, in which the compound having a temperature at onset of melting at least equal to about 100°C is present in an amount of 10-30 % by weight in the composition.

8. Composition according to one of the preceding claims, comprising, in addition, fatty substances chosen from oils, gums, waxes and/or pasty compounds, hydrocarbon-based and/or silicone-based, optionally volatile.

9. Composition according to Claim 8, in which the fatty substances represent 50-98 % by weight of the composition, preferably 70-90 % by weight.

10. Composition according to one of Claims 1 to 4, in which the wax having a temperature at onset of melting at least equal to about 100°C is the only wax present in the composition.

11. Composition according to one of the preceding claims, comprising a pulverulent phase in a quantity of 0-40 % by weight, preferably 10-25 % by weight.

12. Composition according to one of the preceding claims, provided in the form of a cosmetic composition, and especially in the form of a foundation, a blusher or an eyeshadow, a lipstick or a treatment base for the lips, a treatment cream for the body or the face.

13. Process for preparing a composition according to one of the preceding claims, in which:
• in a first stage, the compound having a temperature at onset of melting at least equal to about 100°C is heated to a temperature T1 at which it melts;
• in a second stage, at least a first part of the other fatty substances is added and mixed at a temperature T2 at which they are molten without being degraded, while maintaining a degree of kneading;
• the kneading of the mixture is continued during at least part of its cooling down to room temperature, the mixing and kneading operations being performed in at least one mixer-extruder.

14. Process according to Claim 13, in which all or part of the constituents other than the fatty substances are added during the first and/or the second stage.

15. Process according to one of Claims 13 to 14, in which the heating operations are performed in a mixer-extruder.

16. Process according to one of Claims 13 to 15, in which the heating, mixing and kneading operations are performed in several extruders.

17. Process according to one of Claims 13 to 16, in which the heating, mixing and kneading operations are performed in a single twin-screw extruder.

## Patentansprüche

1. Extrudierte Zusammensetzung, die in Form einer weichen Paste vorliegt und bei 25°C eine dynamische Viskosität von 3 bis 30 Pa·s aufweist, die mit einem Rotationsviskosimeter CONTRAVES TV bestimmt wird, das mit einem beweglichen Körper 'MS-r4' mit der Frequenz von 60 Hz ausgestattet ist, und die eine Fettphase sowie 2 bis 50 Gew.-% mindestens einer Verbindung mit einer Startschmelztemperatur von mindestens 100°C umfaßt, die unter den Wachsen, den Polymeren, den Kunststoffen und den Gemischen dieser Verbindungen ausgewählt ist.

2. Zusammensetzung nach Anspruch 1, in der diese Verbindung ein Wachs mit einer Startschmelztemperatur von über etwa 110°C ist.

3. Zusammensetzung nach Anspruch 1 oder 2, in der diese Verbindung mindestens ein Polyethylenwachs umfaßt.

4. Zusammensetzung nach einem der vorhergehenden Ansprüche, in der diese Verbindung unter dem Polyethylenwachs 'Polywax 2000T-6' von Petrolite, dem Polyethylenwachs 'PED 191' von Hoechst, dem Polyethylenwachs 'Epolene N-14' von Eastman Kodak und den Gemischen dieser Polyethylenwachse ausgewählt ist.

5. Zusammensetzung nach Anspruch 1, in der diese Verbindung ein Polymer oder ein Kunststoff ist, das bzw. der unter den Polypropylenen, den Polyethylenen, den PVC-Polymeren, den Vinylpolymeren oder Acrylpolymeren, wie dem Polystyrol, den Polyamiden, wie dem Nylon, und den Gemischen dieser Verbindungen ausgewählt ist.

6. Zusammensetzung nach Anspruch 5, in der die Polymere oder Kunststoffe als Pulver oder in mikronisierter Form vorliegen.

7. Zusammensetzung nach einem der vorhergehenden Ansprüche, in der die Verbindung mit einer Startschmelztemperatur von mindestens ungefähr 100°C in einem Mengenanteil von 10 bis 30 Gew.-% in der Zusammensetzung vorliegt.

8. Zusammensetzung nach einem der vorhergehenden Ansprüche, die ferner Fettsubstanzen umfaßt, die unter den gegebenenfalls flüchtigen, kohlenwasserstoffhaltigen und/oder siliconhaltigen Ölen, Gummen, Wachsen und/oder pastenförmigen Verbindungen ausgewählt sind.

9. Zusammensetzung nach Anspruch 8, in der die Fettsubstanzen einen Mengenanteil von 50 bis 98 Gew.-% der Zusammensetzung und vorzugsweise von 70 bis 90 Gew.-% ausmachen.

10. Zusammensetzung nach einem der Ansprüche 1 bis 4, in der das Wachs mit einer Startschmelztemperatur von mindestens etwa 100°C das einzige in der Zusammensetzung vorliegende Wachs ist.

11. Zusammensetzung nach einem der vorhergehenden Ansprüche, die eine pulverförmige Phase in einem Mengenanteil von Null bis 40 Gew.-% und vorzugsweise von 10 bis 25 Gew.-% umfaßt.

12. Zusammensetzung nach einem der vorhergehenden Ansprüche, die in Form einer kosmetischen Zusammensetzung und insbesondere in Form eines Make-ups, eines Rouges oder Lidschattens, eines Lippenstifts oder einer Pflegegrundlage für die Lippenpflege, oder auch einer Pflegecreme für den Körper oder das Gesicht vorliegt.

13. Verfahren zur Herstellung einer Zusammensetzung nach einem der vorhergehenden Ansprüche, bei dem:
- in einem ersten Schritt die Verbindung mit einer Startschmelztemperatur von mindestens etwa 100°C auf eine Temperatur T1 erwärmt wird, bei der sie schmilzt,
- in einem zweiten Schritt mindestens ein erster Teil weiterer Fettsubstanzen bei einer Temperatur T2, bei der diese Fettsubstanzen geschmolzen sind, ohne verändert zu sein, zugegeben und untergemischt wird, wobei das Gemisch während dieses Schrittes geknetet wird,
- das Gemisch während mindestens eines Teils der zu seiner Abkühlung auf Umgebungstemperatur erforderlichen Zeitspanne weiter geknetet wird
und
- die Arbeitsgänge des Mischens und Knetens in mindestens einem Mischextruder durchgeführt werden.

14. Verfahren nach Anspruch 13, bei dem alle oder ein Teil der Bestandteile, die von den Fettsubstanzen verschieden sind, bei dem ersten und/oder dem zweiten Schritt zugegeben werden.

15. Verfahren nach Anspruch 13 oder 14, bei dem die Arbeitsgänge des Erwärmens in einem Mischextruder durchgeführt werden.

16. Verfahren nach einem der Ansprüche 13 bis 15, bei dem die Arbeitsgänge des Erwärmens, Mischens und Knetens in mehreren Extrudern durchgeführt werden.

17. Verfahren nach einem der Ansprüche 13 bis 16, bei dem die Arbeitsgänge der Erwärmens, Mischens und Knetens in einem einzigen Zweischneckenextruder durchgeführt werden.
